# EUROPEAN PATENT SPECIFICATION

(11) **EP 2 515 845 B1**
(45) Date of publication and mention of the grant of the patent: **30.04.2014**
(21) Application number: 10763892.6
(22) Date of filing: 22.09.2010
(51) Int. Cl.: A61K 9/00, A61K 31/167, A61K 31/35, A61K 31/46, A61K 31/47, A61P 11/06

(54) **DRY POWDER COMBINATION OF TIOTROPIUM, FORMOTEROL AND A CROMOGLICIC ACID DERIVATIVE**
PULVERZUSAMMENSETZUNG VON TIOTROPIUM, FORMOTEROL UND EINEM CROMOGLICINSÄUREDERIVAT
FORMULATION DE POUDRE SÈCHE COMPRENANT DU TIOTROPIUM, DU FORMOTEROL ET UN DERIVE D'ACIDE CROMOGLICINE

(30) Priority: 25.12.2009 TR 200909790
(43) Date of publication of application: 31.10.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000187
(87) International publication number: WO 2011/078816

(56) References cited:
- WO-A1-00/07567
- WO-A1-00/47200
- DE-A1- 19 835 346
- PAUWELS ET AL: "The current place of nedocromil sodium in the treatment of asthma" JOURNAL OF ALLERGY AND CLINICAL IMMUNOLOGY, MOSBY, INC, US, vol. 98, no. 5, 1 November 1996 (1996-11-01), pages S151-S156, XP005178462 ISSN: 0091-6749

## Description

The present invention relates to a pharmaceutical composition containing tiotropium combined with a β-2 adrenergic receptor antagonist which is formoterol, and an effective amount of a cromolyn derivative which is used for the treatment of respiratory disorder by inhalation route.

Tiotropium (Formula I) is an anticholinergic agent with chemical name (1R, 2R, 4S, 5S, 7S)-7-[(hydroxidi-2-thienylacetyl)oxy]-9,9-dimethyl-3-oxa-9-azoniatricyclo [3.3.1.0^{2,4}] nonane.

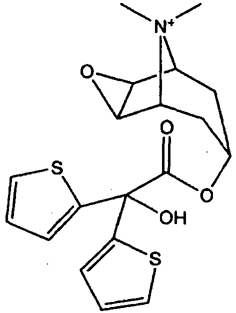

Tiotropium is separated slowly from M1 and M3 receptors that cause broncho-construction, and it is separated rapidly from M2 receptors that inhibit release of acetylcholine from cholinergic nerve endings. This situation occurred in lung receptors demonstrates long acting bronchodilator activity of the drug.

Tiotropium is described in European patent application EP0418716 for the first time. The patent relates to processes for preparing tiotropium, pharmaceutical compositions containing tiotropium, long-acting, strong anticholinergic activity of tiotropium and use of it in the treatment of respiratory disorders.

Use of tiotropium combined with formoterol which is a long acting β-2 adrenergic receptor antagonist is described in WO200047200. According to this patent, when tiotropium bromide is used as combination with formoterol, it is stated that an unexpected therapeutic benefit occurs. Inhalation route is a commonly preferred treatment method for respiratory disorders especially chronic disorders such as asthma and chronic obstructive pulmonary disorder (COPD) which threaten a large portion of the society. The reason is that drug reaches directly and rapidly to target area, accordingly lower doses of drug show desired effect by comparison with doses required for use via oral or parenteral route; and drug which is used in lower doses, show less side-effect than the drug administered via oral and parenteral route.

Lactose, which is a disaccharide, is generally used as a carrier in dry powder formulations developed for use via inhalation,. The amount of lactose is used usually in the range of 10 mg to 50 mg, which is approximately 500-2500 times more than the amount of active agent, for a single dose. However, a large amount of lactose used as a carrier in dry powder formulation caused side effects such as coughing, throat irritation, etc. Additionally, 15 to 25% of each dose of the drugs administered by inhalation can reach the target area, the rest is mostly swallowed. Therefore, dry powder formulations containing large amount of lactose when used by patient with allergy and/or lactose intolerance, causes symptoms such as nausea, stomach cramps, overfullness of the stomach, swelling of stomach, flatus, diarrhea, hives plaque. Because of these drawbacks, dry powder formulations containing tiotropium and formoterol or pharmaceutically acceptable salts, hydrates, enantiomers, racemates, free base, polymorphs, amorphous and/or crystal forms , esters thereof, need to be developed in terms of efficiency and safety.

Salts of cromoglicic acid and nedocromil are used for the treatment of bronchospasm in pharmacology. Because of this property of it, it is used for the treatment of asthma, allergic rhinitis, allergic and vernal conjunctivitis diseases. Products which are marketed by the trademarks of "Intal" and "Cromohexal" provide 20 mg sodium cromoglicate per dose.

The present invention relates to use of salts of cromoglicic acid and/or nedocromil (K) in dry powder formulations containing tiotropium or pharmaceutically acceptable salts thereof (T) combined with formoterol or pharmaceutically acceptable salts thereof (F) administered by inhalation for the treatment of respiratory diseases. Initially, for the purpose of reducing the amount of lactose and alleviation of side effects caused by lactose, K has been added to the dry powder formulation which includes (T) and (F). But surprisingly, it has been found that when (K) is used in an effective amount in the dry powder formulation which includes (T) and (F), a synergistic effect occurs. Therefore, according to the present invention, when salts of cromoglicic acid and/or nedocromil (K) are used in dry powder formulations which include tiotropium or pharmaceutically acceptable salts thereof (T) combined with formoterol or pharmaceutically acceptable salts thereof (F), both required amount of carrier is reduced thus the side-effects caused by carrier are alleviated, and a synergistic effect is obtained for the treatment of respiratory diseases.

The present invention provides a medicament composition containing tiotropium or pharmaceutically acceptable derivatives thereof combined with formoterol or pharmaceutically acceptable derivatives thereof as well as salt of cromoglicic acid and/or nedocromil in an effective amount, for use in the treatment of respiratory diseases.

In the present invention, tiotropium is called as "T" and represents tiotropium or pharmaceutically acceptable salts, hydrates, solvates, esters, polymorphs, free base, crystal and/or amorph forms thereof. In the present invention, formoterol is called as "F" and represents formoterol or pharmaceutically acceptable salts, enantiomers, rasemates, hydrates, solvates, esters, free base, polymorphs, crystal and/or amorph forms thereof. In the present invention, salt of cromoglicic acid and/or nedocromil is called "K" and represents pharmaceutically acceptable salts of cromoglicic acid and/or nedocromil.

The medicament composition in accordance with invention optionally contains a carrier in addition to the substances mentioned above.

In another aspect, the invention provides use of (T), (F) and (K) defined hereinbefore in effective amounts for the preparation of a medicament which is used for the treatment of respiratory diseases especially asthma, chronic obstructive pulmonary dieases and allergic diseases.

In an embodiment of the invention, tiotropium (T) may be in the form of a pharmaceutially acceptable salt, hydrate, solvates ester, polymorph, free base, crystal and/or amorphous form thereof, preferably, tiotropium bromide is used. All of crystal and amorphous forms of tiotropium, which shows different polymorphic forms, can be used within the scope of invention. Also, tiotropium used within the scope of invention can be in the form of anhydrate and hydrate, preferably, tiotropium bromide anhydrate is used.

In an embodiment of the invention, formoterol (F) is in a form selected from a group comprising its pharmaceutically acceptable enantiomers, salts, hydrates, solvates, esters, polymorphs, free base, crystal and amorph forms. Preferably formoterol fumarate, most preferably, RR enantiomer of formoterol fumarate with a purity of at least 85% is used.

In an embodiment of the invention, salt of cromoglicic acid and/or nedocromil (K) can be selected from pharmaceutically acceptable salts thereof. Preferably, it can be either sodium cromoglicate and/or nedocromil sodium.

Dry powder formulation in accordance with the present invention administered via inhalation route optionally contains pharmaceutically acceptable carrier. The carrier can be selected from a group comprising arabinose, glucose, fructose, ribose, mannose, sucrose, trehalose, lactose, maltose, starches, dextran, sugar alcohols such as mannitol and saccharides.

The medicament composition in accordance with the present invention is in the form of micronized dry powder particles. The active agents present in said medicament composition have average particle size of 1 to 20 µm, preferably from 1 to 6 µm. The carrier present in said medicament composition typically has average particle size of not more than 300 µm, preferably not more than 210 µm. Salt of cromoglicic acid and/or nedocromil present in said medicament composition both as an active agent and as an excipient, that has average particle size in the range of 1 to 20 µm and the range of 10 to 300 µm.

Additionally, it is important to provide the inhalation of dry powder formulation in accordance with the present invention effectively and adequately. In the prior art, various devices and methods have been developed in order to provide adequate and effective delivery of dry powder formulations to the patients. While the medicament composition of the present invention was used, inventors encountered a problem of providing an effective amount of dose to the lungs which is a problem widely encountered during inhalation of dry powder formulations. In the prior art, various methods and devices are developed for dealing with this problem.

According to the present invention, it is found that the most suitable method for delivery of effective amount of dose to target area which is lung of the patient and for making patient bring about inhalation in a most suitable way is to inhale said medicament composition as dry powder form and to inhale said medicament from a peelable blister strip.

It is found that compared to the other methods for delivery of dry powder formulation comprising inhalation of dry powder formulation containing (T), (F), and (K) in effective amounts from capsule or from reservoir or from separate blister strips each of which contains a kind of active agent, the method for delivery said dry powder formulation from a peelable blister strip minimize the drawbacks caused by adhesion force of micronized dry powder and the rate of medicament which reaches lungs increases.

In another aspect, the present invention provides a method comprising delivery of medicament composition containing (T), (F) and (K) in effective amounts and optionally a pharmaceutically acceptable carrier from the peelable blister strip by using dry powder inhaler for the treatment of respiratory diseases especially allergic disease.

The cavity volume of each blister in the peelable blister strip contained in the dry powder inhaler which is used for delivery of said medicament composition to the lung, is in the range of 20 to 30 mm³, preferably of 21 to 25 mm³, most preferably of 22 to 23 mm³.

A lid sheet and a base sheet of said blister strip are closed very tightly to provide impermeability by using suitable method.

According to the present invention, the lid sheet or the base sheet of the peelable blister strip consists of three layers. Two of these layers are polymeric layers and the other one is aluminium foil. Aliminium foil is used in both the lid sheet and the base sheet of the peelable blister strip to provide high humidity and gas protection because of that aluminium foil is conventionally used in both the lid sheet and the base sheet of blister strip with high humidty and gas protection. These layers must have the sufficient thickness which provides the protection for the stability of humidity sensitive dry powder formulation which is stored in blister cavity. Because of this reason, the thickness of aluminium foil that is used in the lid sheet and the base sheet of the blister strip is in the range of 10 to 40 µm, preferably of 15 to 30 µm.

Two of the layers contained by the lid sheet and the base sheet of the peelable blister strip according to the present invention are polymeric layers. These polymeric layers may be made from either same or different polymers. The thickness of these polymeric layers depends on the type of polymeric substance used and its properties. Therefore, the thickness of each polymeric layer which is used in the lid sheet and the base sheet of said blister strip is in the range of 15 to 60 µm, preferably of 20 to 35 µm depending on the type of used polymer.

The inside layer of blister cavity of said blister strip which is in contact with dry powder formulation is polymeric layer because of the fact that aluminium foil causes adhesion of a part of dry powder formulation to inside layer of the cavity due to electrostatic forces, and hence cause uncontrolled dosing.

According to the present invention, the polymers used for forming polymeric layers are preferably selected from a group comprising thermo-plastic polymers such as polyethylene, polypropylene, polystyrene, polyolefin, polyamide, polyvinyl chloride, polyurethane.

The layers which are used for making up the lid sheet and the base sheet of the peelable blister strip in accordance with present invention, are preferably same for each sheet, however the polymeric substances used for forming polymeric layers are preferably different from each other. Moreover, each of the blister cavities which constitute the peelable blister strip, can be different in shape as long as it has the properties defined above.

The devices used to inhale the dry powder formulation in accordance with the present invention may be multi dose inhalers present in the prior art. For this reason, the invention provides a medicament composition as it is mentioned before. In another aspect, the invention provides a method for delivery of medicament composition to patient's lungs effectively as it is mentioned before.

The medicament composition in accordance with the present invention containing active agents which is preferably in dry powder form is stored in the peelable blister strip and during inhalation 2 to 50 milligram of said medicament composition is delivered to patients by using a multi dose inhaler, after each movement of the device.

In the medicament composition of present invention T is present in the amount of 1 to 50 µg, preferably 5 to 30 µg, F is present in the amount of 1 to 50 µg, preferably 5 to 25 µg, K is present in the amount of 1 to 50 mg, preferably 1 to 25 mg. Accordingly, the ratio of the total weight of T and F to the weight of K in said medicament composition is in the range of 1:100 to 1:1800. Additionally, in medicament composition pharmaceutically acceptable carrier can be used for the purpose of adjusting each dose of medicament composition which is in dry powder form to the range of 2 to 50 mg.

Medicament composition in accordance with present invention can be used for the treatment of many respiratory diseases such as asthma, chronic obstructive pulmonary disorder (COPD) and allergic rhinitis. Accordingly, the respiratory diseases include but are not restricted to; allergic or non-allergic asthma in various phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition to this, the medicament composition in accordance with the present invention is used especially for the symptomatic treatment of asthma, allergic rhinitis and COPD.

This present invention, is explained with examples given below, but it is not restricted to these examples. Parts given in examples represent the weights of ingredients.

### Example 1

The medicament composition in dry powder form which is stored in a peelable blister strip to be delivered by using multi-dose inhaler and is prepared by mixing 18 parts of tiotropium bromide anhydrate, 12 parts of formoterol fumarate and 2000 parts of sodium cromoglicate, having average particle size of 1 to 5 µm, and 12000 parts of sodium cromoglicate having average particle size of not more than 300 µm, which are micronized in an air jet mill, and 2000 parts of lactose as second carrier.

As given in the following example, tiotropium bromide anhydrate (T) may be extended tiotropium or pharmaceutically acceptable salts, hydrates, solvates, esters, polymorphs, amorph and/or crystal forms thereof; formoterol fumarate (F) may be extended formoterol or pharmaceutically acceptable salts, enantiomers, racemates, hydrates, solvates, esters ,polymorphs, amorph and/or crystal forms thereof, sodium cromoglicate (K) may be extended salts of cromoglicic acid and pharmaceutically acceptable salts of nedocromil, more or less amount of pharmaceutically acceptable carrier may optionally be added, and, thus example 1 is repeated as following:

| | | Amount of Tiotropium Bromide | Amount of Formoterol Fumarate | Amount of A salt of Cromoglicic Acid (<300 µm) | Amount of A salt of Cromoglicic Acid (1-5 µm) | Amount of Carrier (<300 µm) |
|---|---|---|---|---|---|---|
| | | parts | parts | parts | parts | parts |
| Example | 2 | 18 | 12 | 2000 | 10000 | 2000 |
| Example | 3 | 18 | 12 | 4000 | 10000 | 0 |
| Example | 4 | 18 | 12 | 4000 | 11000 | 2000 |
| Example | 5 | 18 | 12 | 2000 | 12000 | 0 |
| Example | 6 | 18 | 12 | 4000 | 12000 | 0 |
| Example | 7 | 18 | 12 | 4000 | 12000 | 2000 |
| Example | 8 | 9 | 12 | 2000 | 13000 | 0 |
| Example | 9 | 9 | 12 | 2000 | 12000 | 2000 |
| Example | 10 | 9 | 12 | 4000 | 10000 | 0 |
| Example | 11 | 9 | 12 | 4000 | 11000 | 2000 |
| Example | 12 | 9 | 12 | 2000 | 15000 | 0 |
| Example | 13 | 9 | 12 | 4000 | 11000 | 0 |
| Example | 14 | 9 | 12 | 2000 | 10000 | 2000 |
| Example | 15 | 9 | 12 | 4000 | 11000 | 2000 |
| Example | 16 | 18 | 6 | 2000 | 12000 | 2000 |
| Example | 17 | 18 | 6 | 2000 | 13000 | 0 |
| Example | 18 | 18 | 6 | 4000 | 10000 | 2000 |
| Example | 19 | 18 | 6 | 4000 | 10000 | 0 |
| Example | 20 | 18 | 6 | 2000 | 15000 | 2000 |
| Example | 21 | 18 | 6 | 2000 | 10000 | 0 |
| Example | 22 | 18 | 6 | 4000 | 12000 | 0 |
| Example | 23 | 18 | 9 | 4000 | 12000 | 2000 |
| Example | 24 | 9 | 9 | 2000 | 10000 | 2000 |
| Example | 25 | 9 | 9 | 2000 | 10000 | 0 |
| Example | 26 | 9 | 9 | 4000 | 12000 | 2000 |
| Example | 27 | 9 | 9 | 4000 | 12000 | 0 |
| Example | 28 | 9 | 9 | 2000 | 15000 | 2000 |
| Example | 29 | 9 | 9 | 2000 | 12000 | 0 |
| Example | 30 | 9 | 9 | 4000 | 13000 | 2000 |
| Example | 31 | 9 | 9 | 4000 | 14000 | 0 |

## Claims

1. A medicament composition containing combination of tiotropium (T) and formoterol (F) for use in the treatment of respiratory disorder by inhalation route wherein said medicament composition contains an effective amount of a pharmaceutically acceptable salt of cromoglicic acid and/or nedocromil (K) **characterized in that** said salt of cromoglicic acid and/or nedocromil is present in said medicament composition both as an active agent and as an excipient and has average particle size in the range of 1 to 6 µm and in the range of 10 to 300 µm.

2. A medicament composition for use according to claim 1, wherein tiotropium (T) is selected from a group comprising pharmaceutically acceptable salts, hydrates, solvates, esters, polymorphs, crystalline or amorphous forms thereof.

3. A medicament composition for use according to claim 2, wherein tiotropium (T) is preferably tiotropium bromide.

4. A medicament composition for use according to claim 1, wherein tiotropium (T) is preferably tiotropium bromide anhydrate.

5. A medicament composition for use according to claim 1, wherein formoterol (F) is selected from a group comprising pharmaceutically acceptable salts, enantiomers, racemates, hydrates, solvates, esters, polymorphs, crystals or amorphous forms thereof.

6. A medicament composition for use according to claim 5, wherein formoterol (F) is preferably RR formoterol fumarate.

7. A medicament composition for use according to any one of the preceding claims, wherein salt of cromoglicic acid (K) is sodium cromoglicate.

8. A medicament composition for use according to any one of claims 1 to 7, wherein salt of nedocromil (K) is nedocromil sodium.

9. A medicament composition for use according to claim 1, wherein said medicament composition used locally for the treatment of respiratory diseases by inhalation route contains micronized dry powder

10. A medicament composition in dry powder form for use according to claim 9, wherein said medicament composition administered directly to airways by using a dry powder inhalation device.

11. A medicament composition for use according to claim 9, wherein tiotropium (T) and formoterol (F) have average particle size of 1 to 6 µm.

12. A medicament composition in dry powder form for use according to any one of the preceding claims, wherein tiotropium (T) is present in the amount of 1 to 50 µg, formoterol (F) is present in the amount of 1 to 50 µg and salt of cromoglicic acid and/or nedocromil (K) is present in the amount of 1 to 50 mg for each dose.

13. A medicament composition in dry powder form for use according to claim 11, wherein the ratio of the total weight of tiotropium (T) and formoterol (F) to amount of a salt of cromoglicic acid (K) is in the range of 1:100 to 1:1800.

14. A medicament composition in dry powder form for use according to claim 8, wherein said medicament optionally contains a pharmaceutically acceptable carrier.

15. A medicament composition for use according to claim 14 wherein said medicament composition containing a pharmaceutically acceptable carrier selected from a group comprising monosaccharide, disaccharide, polysaccharide and oligosaccharide.

## Patentansprüche

1. Eine Arzneimittelmischung, die eine Kombination aus Tiotropium (T) und Formoterol (F) enthält und bei der Behandlung von Atemwegeserkrankungen durch Inhalation eingesetzt wird, wobei besagte Medikamentenmischung eine wirksame Menge von pharmazeutisch unbedenklichen Salzen der Cromoglicinsäure und/oder von Nedokromil (K) enthält, gekennzeichnet in diesem Salz der Cromoglicinsäure und/oder Nedokromil in besagter Arzneimittelinischung sowohl als Wirkstoff und als Hilfsstoff vorliegt und eine durchschnittliche Größe von 1 bis 6 µm und einen Bereich von 10 bis 300 µm aufweist.

2. Eine Arzneimittelmischung für einen unter Anspruch 1 beschriebenen Einsatz, wobei Tiotropium (T) aus einer Gruppe von pharmazeutisch unbedenklichen Salzen, Hydraten, Lösungsmitteln, Estern und polymorphen, kristallinen und amorphen Formen davon ausgewählt wird.

3. Eine Arzneimittelmischung für einen unter Anspruch 2 beschriebenen Einsatz, wobei Tiotropium (T) vorzugsweise Tiotropium-Bromid ist.

4. Eine Arzneimittelmischung für einen unter Anspruch 1 beschriebenen Einsatz, wobei Tiotropium (T) vorzugsweise Tiotropium-Bromid-Anhydrat ist.

5. Eine Arzneimittelmischung für einen unter Anspruch 1 beschriebenen Einsatz, wobei Formoterol (F) aus einer Gruppe von pharmazeutisch unbedenklichen Salzen, Enantiomeren, Racemates, Hydraten, Lösungsmitteln, Estern und polymorphen, kristallinen und amorphen Formen davon ausgewählt wird.

6. Eine Arzneimittelmischung für einen unter Anspruch 5 beschriebenen Einsatz, wobei Formoterol (F) vorzugsweise RR-Formoterol-Fumarat ist.

7. Eine Arzneimittelmischung für einen unter den zuvor genannten Ansprüchen beschriebenen Einsatz, wobei Salze der Cromoglicinsäure (K) Sodium-Cromoglicat ist.

8. Eine Arzneimittelmischung für einen unter Ansprüchen 1-7 beschriebenen Einsatz, wobei das Salz von Nedokromil (K) Nedokromit-Sodium ist.

9. Eine Arzneimittelmischung für einen unter Anspruch 1 beschriebenen Einsatz, wobei besagte Arzneimittelmischung lokal zur Behandlung von Aternwegserkrankungen durch Inhalation eingesetzt wird und mikronisiertes Trockenpulver enthält.

10. Eine Arzneimittelmischung in Trockenpulverform für einen unter Anspruch 9 beschriebenen Einsatz, wobei besagte Arzneimittelmischung unter Verwendung eines Inhalationsgerätes für Trockenpulver direkt in die Atemwege verabreicht wird.

11. Eine Arzneimittelmischung für einen unter Anspruch 9 beschriebenen Einsatz, wobei Tiotropium (T) und Formoterol (F) eine durchschnittliche Partikelgröße zwischen 1 und 6 µm besitzt.

12. Eine Arzneimittelmischung in Trockenpulverform für einen unter den zuvor genannten Ansprüchen beschriebenen Einsatz, wobei Tiotropium (T) in einer Menge zwischen 1 und 50 µg vorliegt, Formoterol (F) in einer Menge zwischen 1 und 50 µg und Salze der Cromoglicinsäure und/oder Nedokromil (K) in einer Menge zwischen 1 und 50 µg für jede Dosis vorliegt.

13. Eine Arzneimittelmischung in Trockenpulverform für einen unter Anspruch 11 beschriebenen Einsatz, wobei sich das Verhältnis zwischen Gesamtgewicht von Tiotropium (T) und Formoterol (F) und der Menge von Salzen der Cromoglicinsäure (K) zwischen 1:100 und 1:1800 verhält.

14. Eine Arzneimitielmischung in Trockenpulverform für einen unter Anspruch 8 beschriebenen Einsatz, wobei das Medikament optional einen pharmazeutisch unbedenklichen Träger enthält.

15. Eine Armeimittelmischung für einen unter Anspruch 14 beschriebenen Einsatz, wobei das Medikament einen pharmazeutisch unbedenklichen Träger enthält, das aus einer Gruppe von Monosacchariden, Disacchariden, Polysacchariden und Oligosacchariden ausgewählt wird.

## Revendications

1. Composition médicamenteuse contenant une combinaison de tiotropium (T) et de formotérol (F), utilisée pour le traitement des troubles respiratoires et administrée par inhalation. Elle contient également une quantité suffisante pharmaceutiquement acceptable de sel d'acide cromoglicique et/ou de nédocromil (K), **caractérisée par** la présence dudit sel d'acide cromoglicique et/de nédrocromil dans ladite composition médicamenteuse, à la fois comme agent actif et comme excipient et dont la taille moyenne des particules est comprise entre 1 et 6 µm et entre 10 et 300 µm.

2. Composition médicamenteuse utilisée selon la revendication n° 1, dans laquelle le tiotropium (T) est choisi dans un groupe comprenant des sels, des hydrates, des solvates, des esters, des polymorphes, de la cristalline pharmaceutiquement acceptables, ou des formes amorphes.

3. Composition médicamenteuse utilisée selon la revendication n° 2, dans laquelle le tiotropium (T) est de préférence le bromure de tiotropium.

4. Composition médicamenteuse utilisée selon la revendication n° 1, dans laquelle le tiotropium (T) est de préférence l'anhydre du bromure de tiotropium.

5. Composition médicamenteuse utilisée selon la revendication n° 1, dans laquelle le formotérol (F) est choisi dans un groupe comprenant des sels, des énantiomères, des racémates, des hydrates, des solvates, des esters, des polymorphes, des cristaux ou des formes amorphes pharmaceutiquement acceptables.

6. Composition médicamenteuse utilisée selon la revendication n° 5, dans laquelle le formotérol (F) est de préférence le formotétrol fumarate de type RR.

7. Composition médicamenteuse utilisée selon l'une des revendications énumérées ci-dessus, dans laquelle le sel d'acide cromoglicique (K) est le cromoglycate de sodium.

8. Composition médicamenteuse utilisée selon l'une des revendications n° 1 à 7, dans laquelle le sel de nédocromil (K) est le nédocromil de sodium.

9. Composition médicamenteuse utilisée selon la revendication n° 1, dans laquelle ladite composition médicamenteuse, destinée à une utilisation locale pour le traitement des maladies respiratoires et administrée par inhalation, contient de la poudre sèche micronisée.

10. Composition médicamenteuse sous la forme d'une poudre sèche utilisée selon la revendication n° 9, dans laquelle ladite composition médicamenteuse est administrée directement par voies respiratoires, à l'aide d'un appareil d'inhalation de poudre sèche.

11. Composition médicamenteuse utilisée selon la revendication n° 9, dans laquelle les particules de tiotropium (T) et de formotérol (F) ont une taille moyenne de 1 à 6 µm.

12. Composition médicamenteuse sous la forme d'une poudre sèche, utilisée selon l'une des revendications énumérées ci-dessus, dans laquelle la quantité de tiotropium est de 1 à 50 µg, celle du formotérol de 1 à 50 µg et celle du sel d'acide cromoglicique et/ou du nédocromil (K) de 1 à 50 mg pour chaque dose.

13. Composition médicamenteuse sous la forme d'une poudre sèche utilisée selon la revendication n° 11, dans laquelle le rapport du poids total du tiotropium (T) et du formotérol (F) sur la quantité d'un sel d'acide cromoglicique (K) est de l'ordre de 1/100 à 1/1800.

14. Composition médicamenteuse sous la forme d'une poudre sèche utilisée selon la revendication n 8, dans laquelle ladite composition médicamenteuse contient en option un vecteur pharmaceutiquement acceptable.

15. Composition médicamenteuse utilisée selon la revendication n 14, dans laquelle ladite composition médicamenteuse contient un vecteur pharmaceutiquement acceptable sélectionné dans un groupe composé de monosaccharide, de disaccharide, de polysaccharide et d'oligosaccharide.
